# EUROPEAN PATENT SPECIFICATION

(11) **EP 4 011 328 B1**
(45) Date of publication and mention of the grant of the patent: **13.09.2023**
(21) Application number: 20212594.4
(22) Date of filing: 08.12.2020
(51) Int. Cl.: A61F 2/24, A61F 2/90, A61F 2/958

(54) **ANNULOPLASTY DEVICE**
ANNULOPLASTIEVORRICHTUNG
DISPOSITIF D'ANNULOPLASTIE

(43) Date of publication of application: 15.06.2022
(73) Proprietor: HVR Cardio Oy, 02600 Espoo (FI)
(72) Inventor: Keränen, Olli, 23741 Bjärred (SE)
(74) Representative: Invent Horizon IP

(56) References cited:
- WO-A1-2005/058206
- US-A1- 2020 281 722
- US-B2- 8 187 324

## Description

### Field of the Invention

This invention pertains in general to the field of annuloplasty devices for treating a defective mitral valve. More particularly the invention relates to an annuloplasty device for treating a defective mitral valve via coronary sinus.

### Background of the Invention

Diseased mitral and tricuspid valves frequently need replacement or repair. The mitral and tricuspid valve leaflets or supporting chordae may degenerate and weaken or the annulus may dilate leading to valve leak. Mitral and tricuspid valve replacement and repair are frequently performed with aid of an annuloplasty ring, used to reduce the diameter of the annulus, or modify the geometry of the annulus in any other way, or aid as a generally supporting structure during the valve replacement or repair procedure.

Implants have previously been introduced into the coronary sinus (CS) in order to affect the shape of the valve annulus and thereby the valve function. WO02/062270 discloses such implant that is aimed to replace annuloplasty rings. Implanting annuloplasty devices in the CS is a procedure that entails several challenges, such as re-shaping the annulus in a manner that sustain proper valve function, and ensuring the correct position of the device in the CS over time. Possible traumatic effects on the CS itself have to be taken into account, as well as the complexity of the implant and the procedure. Prior art devices typically have suboptimal performance in several of the aforementioned aspects of annuloplasty via the CS. A problem is to ensure that a significant part of the annulus is reshaped while providing for atraumatic engagement with the anatomy. A problem with the prior art is complex and difficult-to-operate devices, that may require frequent adjustment and repositioning to ensure the correct function over time. This may have dire consequences for the patient and the health care system. Patient risk is increased.

It is desirable to increase the degree of control of the downsizing procedure, i.e. the re-shaping of the annulus, while ensuring a secure anchoring of the implant and minimal risk of damage to the CS.

Hence, an improved annuloplasty device for performing downsizing and reshaping of the valve annulus would be advantageous and in particular allowing for ensuring long-term functioning, less complex procedure, and less traumatic effects on the anatomy and increased patient safety. Also, a method of downsizing and reshaping the mitral valve annulus with such annuloplasty device would be advantageous.

### Summary of the Invention

Accordingly, examples of the present invention preferably seek to mitigate, alleviate or eliminate one or more deficiencies, disadvantages or issues in the art, such as the above-identified, singly or in any combination by providing a device according to the appended patent claims.

The invention relates to an annuloplasty device for treating a defective mitral valve as defined by independent claim 1. Preferred embodiments are recited in the dependent claims.

Further examples of the invention are defined in the dependent claims.

Some examples of the disclosure provide for long-term functioning of a repaired mitral valve.

Some examples of the disclosure provide for less complex downsizing procedures of the mitral valve.

Some examples of the disclosure provide for improved control of the downsizing procedure of the mitral valve.

Some examples of the disclosure provide for a reduced risk of damaging the anatomy such as the CS.

Some examples of the disclosure provide for a secure downsizing while at the same time reducing the risk of damaging the anatomy such as the CS.

Some examples of the disclosure provide for improved downsizing of the mitral valve annulus while ensuring an atraumatic procedure.

Some examples of the disclosure provide for reduced risk of long-term negative effects of CS implants.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is a schematic illustration of an annuloplasty device, in a cross-sectional view, according to the invention;
Fig. 2a is a schematic illustration of an annuloplasty device, according to the invention;
Fig. 2b is a schematic illustration of the annuloplasty device in Fig. 2a, with an expanded proximal portion, according to the invention;
Figs. 3a-b are schematic illustrations of an annuloplasty device, with different lengths between the proximal expandable portion and the distal anchoring portion, according to one example;
Fig. 4a is a schematic illustration of an annuloplasty device, where a catheter is advanced over an elongated displacement unit, according to one example;
Fig. 4b is a schematic illustration of the annuloplasty device in Fig. 4a, where a stent is advanced over the elongated displacement unit, according to one example;
Fig. 4c is a schematic illustration of the annuloplasty device in Fig. 4b, where the stent is exposed in position over the elongated displacement unit, according to one example;
Fig. 4d is a schematic illustration of the annuloplasty device in Fig. 4c, where the elongated displacement unit is withdrawn through the stent, according to one example;
Fig. 4e is a schematic illustration of the annuloplasty device in Fig. 4d, in a cross-sectional view across the radial direction, according to one example;
Figs. 5a-b are schematic illustrations of an annuloplasty device positioned in the coronary sinus (CS), where an elongated displacement unit has been anchored with proximal expandable portion and a distal anchor, according to one example;
Figs. 5c-d are schematic illustrations of an annuloplasty device, where a catheter has been advanced through the proximal expandable portion and over the elongated displacement unit, according to one example;
Figs. 5e-f are schematic illustrations of an annuloplasty device, where a stent has been advanced in the catheter of Figs. 5c-d, according to one example;
Figs. 5g-h are schematic illustrations of an annuloplasty device, where the stent of Figs. 5e-f has been at least partly expanded in the CS, according to one example;
Fig. 5i is a schematic illustration of an annuloplasty device, where the stent of Figs. 5e-f has been fully expanded in the CS, according to one example;
Fig. 5j is a schematic illustration of an annuloplasty device, where the elongated displacement unit of Fig. 5i has been withdrawn through the stent, according to one example;
Figs. 5k-l are schematic illustrations of an annuloplasty device, where the stent of Figs. 5e-f has been fully expanded and implanted in the CS, according to one example;
Fig. 6a is a schematic illustration of a mitral valve and the adjacent coronary sinus;
Fig. 6b is a schematic illustration of an annuloplasty device, where the stent of Figs. 5e-f has been fully expanded and implanted in the CS to re-shape the annulus, according to one example;
Fig. 7 is a schematic illustration of an annuloplasty device, in a cross-sectional view, according to one example;
Fig. 8a is a flow chart of a method for treating a defective mitral valve according to one example; and
Fig. 8b is a flow chart of a method for treating a defective mitral valve according to one example.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

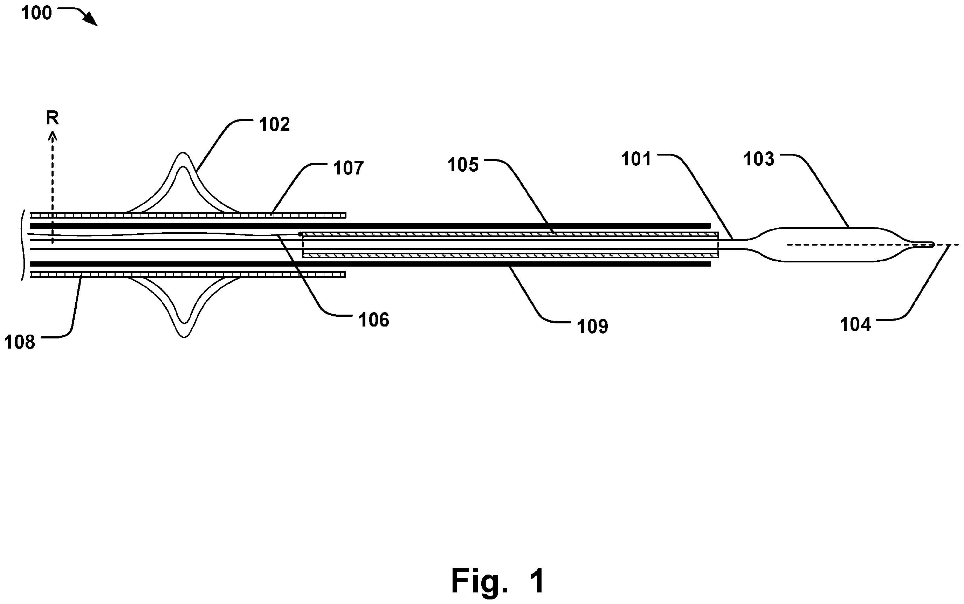

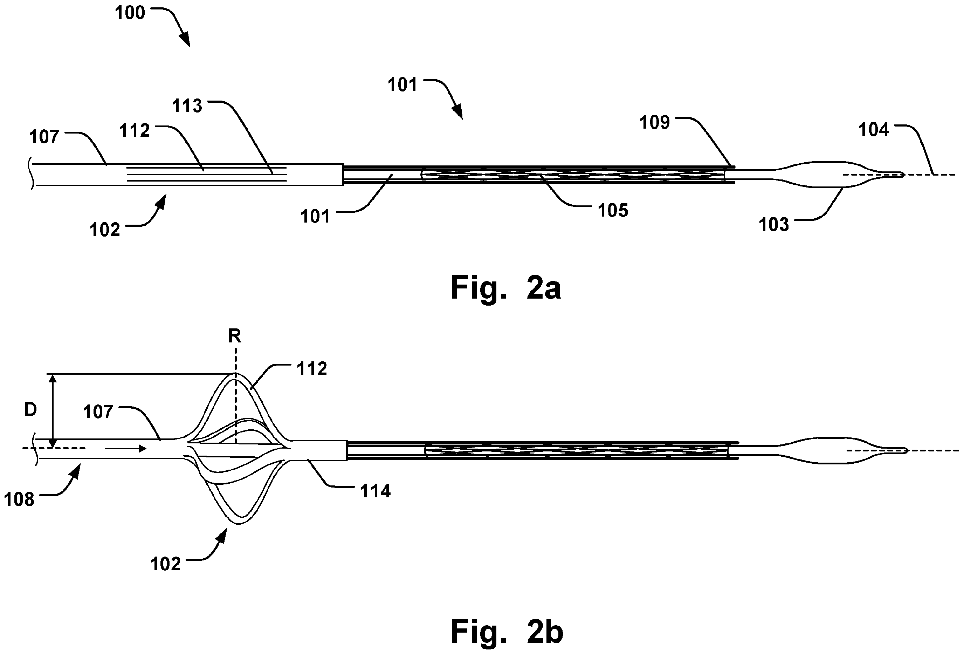

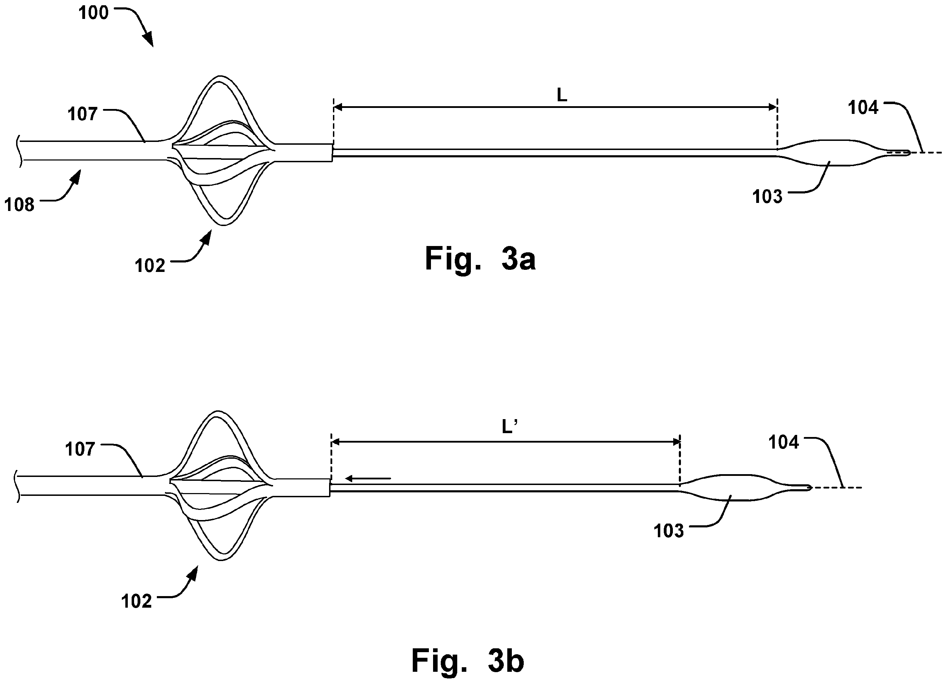

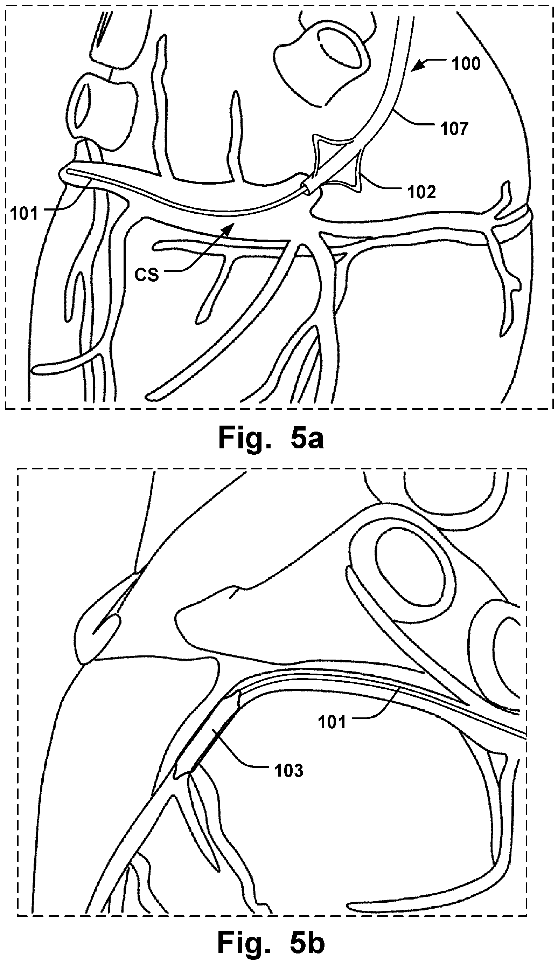

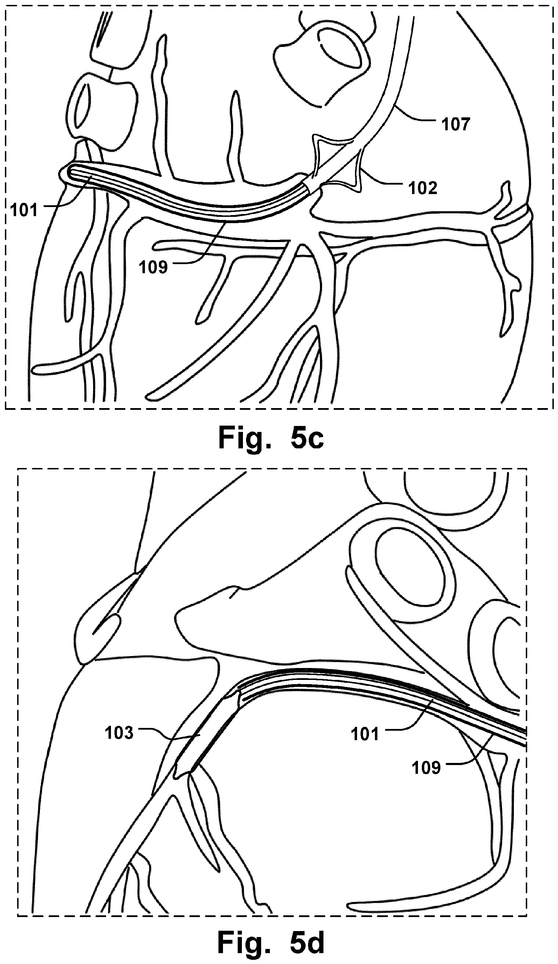

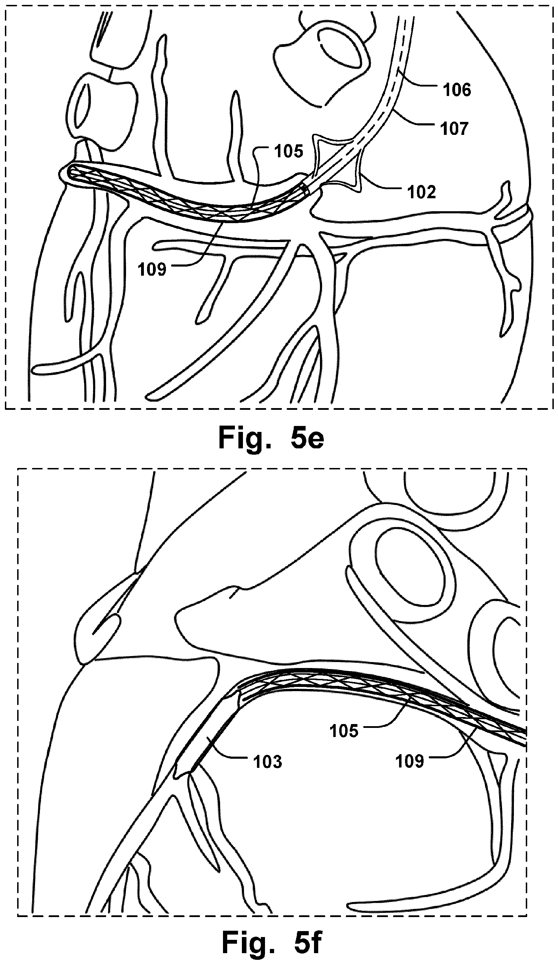

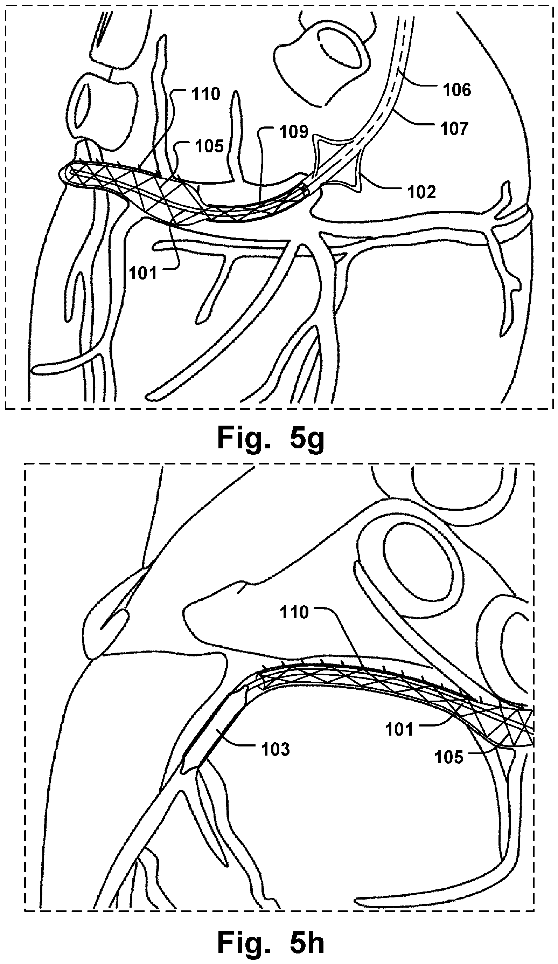

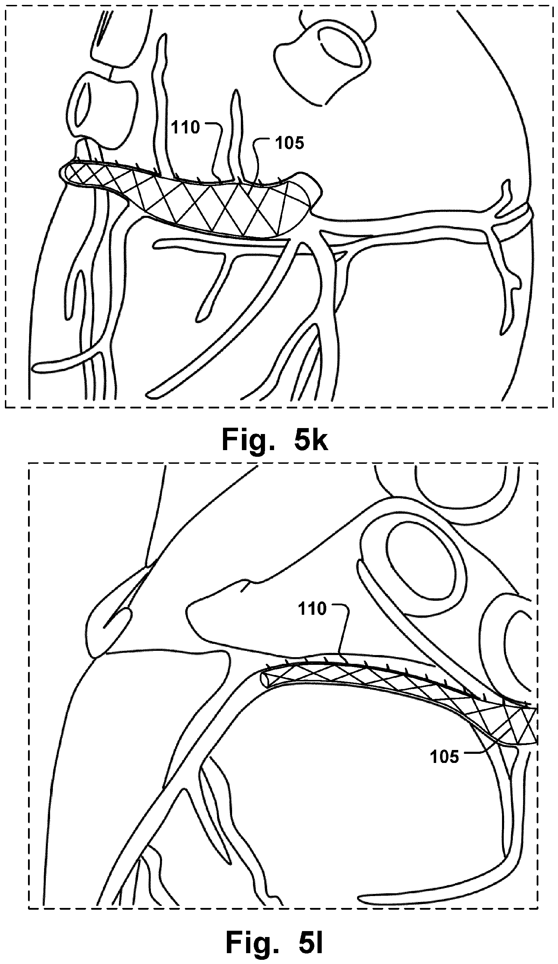

Fig. 1 schematically illustrates an annuloplasty device 100 for treating a defective mitral valve having an annulus. The annuloplasty device 100 comprises a removable and flexible elongate displacement unit 101 for temporary insertion into a coronary sinus (CS) adjacent the mitral valve. The displacement unit 101 has a delivery state for delivery into the CS, and an activated state to which the displacement unit 101 is temporarily and reversibly transferable from said delivery state. The annuloplasty device 100 comprises a proximal reversibly expandable portion 102. The proximal expandable portion 102 is reversibly foldable to an expanded state for positioning against a tissue wall at the entrance of the CS. Figs. 2a-b show an example where the proximal expandable portion 102 moves from a collapsed state in Fig. 2a, to an expanded state in Fig. 2b. Fig. 5a illustrates schematically how the proximal expandable portion 102 is positioned outside the CS, for pushing against the wall at the entrance of the CS. The displacement unit 101 comprises a distal anchoring portion 103 being movable in relation to the proximal expandable portion 102 in a longitudinal direction 104 of the displacement unit 101 to the aforementioned activated state. Fig. 5b illustrates schematically how the distal anchoring portion 103 is anchored in the CS. The distal anchoring portion 103 may be anchored in the great cardiac vein. Figs. 3a-b illustrate an example where the distal anchoring portion 103 is movable such that a distance between the proximal expandable portion 102 and the distal anchoring portion 103 is varied. The distance is reduced from a length denoted L in Fig. 3a to a reduced length denoted L' in Fig. 3b. In the activated state, the annuloplasty device 100, when placed in the CS, modifies the annulus to a modified shape where the annulus is downsized and the leaflets may co-apt. Thus, when the annuloplasty device 100 is placed in the CS as exemplified in Figs. 5a-b, and the distal anchoring portion 103 is anchored, the latter may be withdrawn towards the proximal expandable portion 102 which exerts a counter force against the tissue wall at the entrance of the CS. This allows for re-shaping the annulus of the mitral valve.

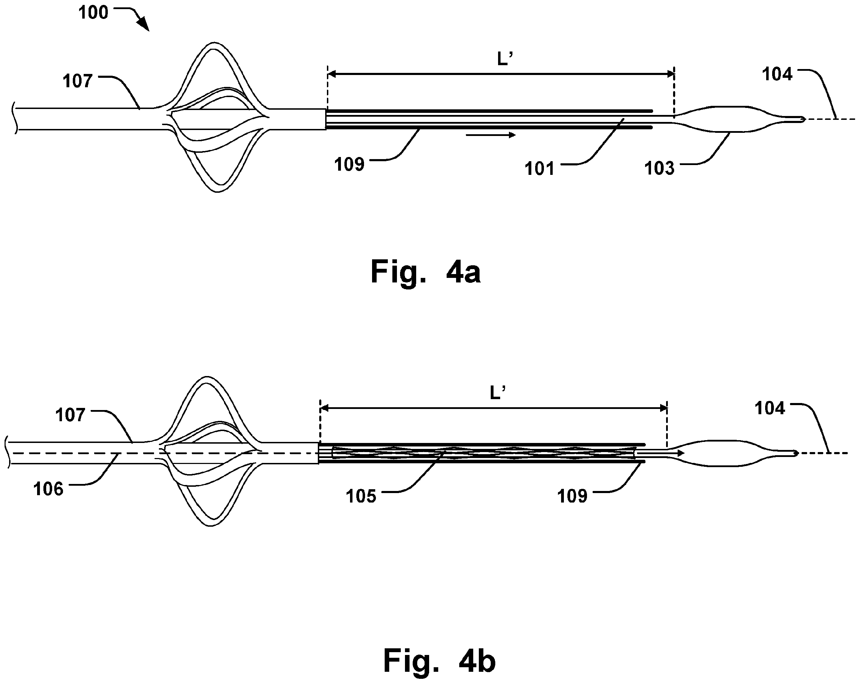

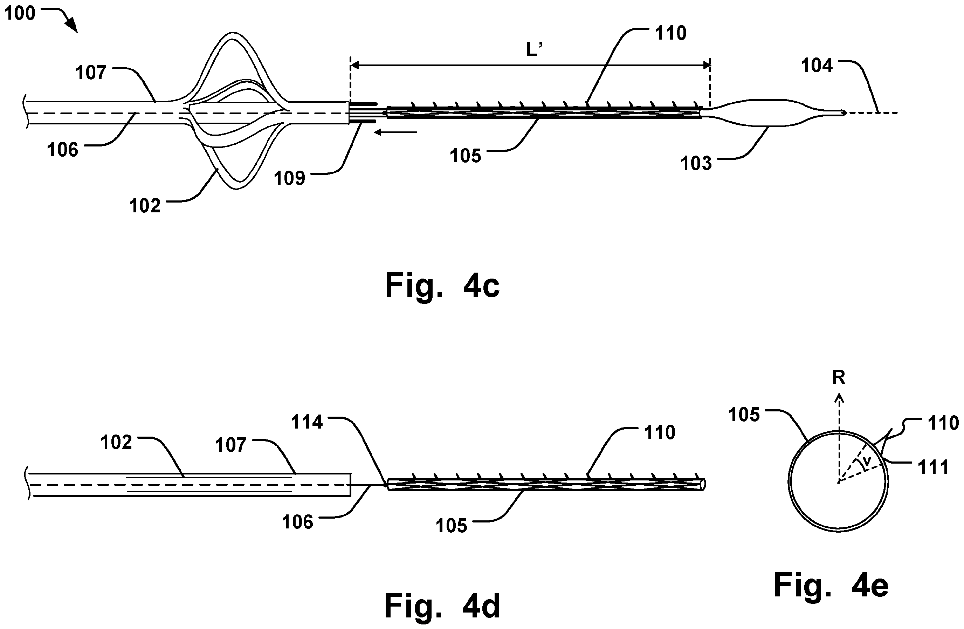

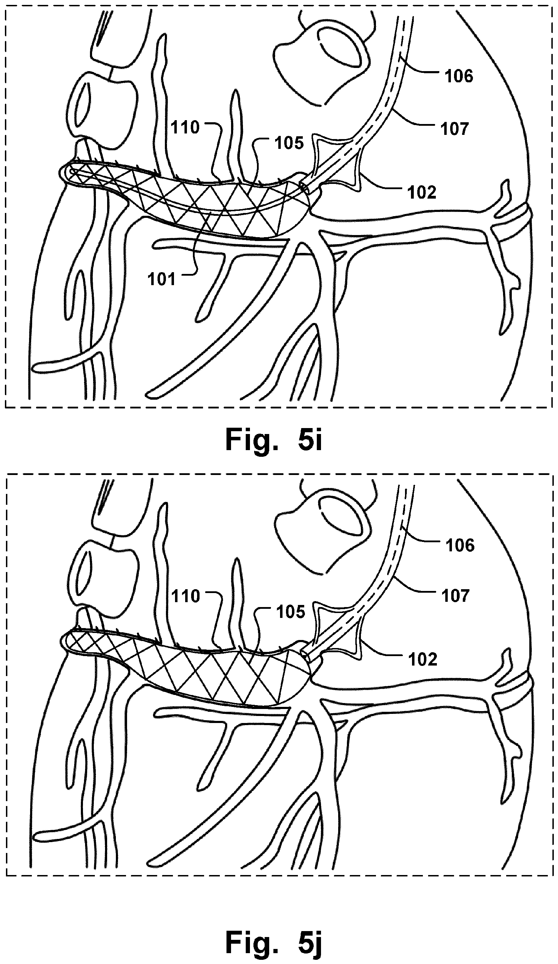

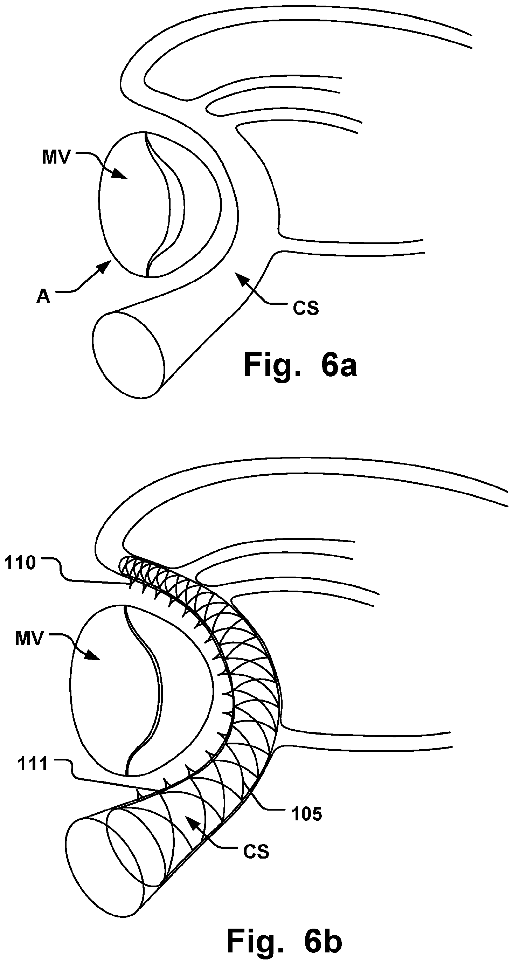

Turning again to Fig. 1, the annuloplasty device 100 comprises a stent 105 arranged around the displacement unit 101 and being movable relative the displacement unit 101 along the longitudinal direction 104 for insertion into the CS. Fig. 4b illustrate schematically how the stent 105 is advanced over the displacement unit 101 towards the distal anchoring portion 103. Figs. 5e-f illustrate the position of the stent 105 over the displacement unit 101 when the annuloplasty device 100 is placed in the CS. The stent 105 may be expanded for anchoring into the CS when the displacement unit 101 has re-shaped the annulus, as illustrated in Figs. 5g-i and described further below. The stent 105 is releasably connected to a delivery device 106 as schematically indicated in Fig. 4d and 5j, and may be released in the CS to maintain the re-shaped form of the annulus after the displacement unit 101 has been withdrawn, as illustrated in Figs. 5j-l, and 6b. Fig. 6a is an illustration of the heart showing the CS in relation to the mitral valve (MV) in a top-down view. The CS lies adjacent the MV and follows a curvature around the annulus (A) of the MV. The stent 105 may have a releasable connection 114 to the delivery device 106 as exemplified in Fig. 4d. It is conceivable that the delivery device 106 may be configured push or pull the stent 105 relative the displacement unit 101 along the longitudinal direction 104. The stent 105 is arranged radially between the displacement unit 101 and the proximal expandable portion 102 in a radial direction (R), as illustrated in e.g. Fig. 1. The radial direction (R) is perpendicular to the longitudinal direction 104. Having the stent 105 arranged between the displacement unit 101 and the proximal expandable portion 102 allows for advancing the stent 105 over the displacement unit 101 into the CS while the proximal expandable portion 102 is expanded at the outside of the CS and the displacement unit 101 is in the activated state. The re-shaping of the annulus may thus be carefully controlled and optimized with the displacement unit 101, by varying the length (L), before the stent 105 is positioned and finally anchored in the CS. Once the leaflets co-apt properly and no regurgitation occurs, e.g. by observing flow characteristics and leaflet movement, the stent 105 may be expanded gradually (Figs. 5g-i), e.g. by withdrawing a catheter 109 arranged over the stent 105, as described further below. Connection to the stent 105 can be maintained with the delivery device 106 after the stent 105 has been fully expanded (Fig. 5i). The force exerted by the displacement unit 101 on the annulus may then be gradually released, e.g. by reducing the tension between the proximal expandable portion 102 and the distal anchoring portion 103. The flow characteristics and leaflet movement may be continuously observed to ensure no regurgitation occurs. The displacement unit 101 can be fully withdrawn through the stent 105 (Fig. 5j), and the stent 105 can be released (Figs. 5k-l) if no regurgitation occurs. Otherwise the stent 105 may be captured, e.g. by advancing a catheter 109 overt the stent 105, and the re-shaping of the annulus may be adjusted further with the displacement unit 101, and/or another stent 105 of a different size may be introduced through the proximal expandable portion 102 and over the displacement unit 101 to repeat the procedure.

It is conceivable that the stent 105 may be advanced through the proximal expandable portion 102 and over the displacement unit 101 into the CS (Figs. 5e-f), before or after the displacement unit 101 has re-shaped the annulus. Regardless, the stent 105 is expanded and fixed in the CS after the re-shaping has been performed.

The annuloplasty device 100 thus provides for a facilitated annuloplasty procedure via the CS. The re-shaping of the annulus can be carefully controlled and optimized with the displacement unit 101 and the stent 105 can be anchored in the CS to maintain the modified shape when the proper valve function can be confirmed. The safety of the procedure is improved as the position of the stent 105 relative the displacement unit 101 and the CS can be varied and optimized while the displacement unit 101 already provides the downsizing effect of the valve in the activated state. The need to introduce complex elements into the implanted device, i.e. the stent, in order to provide downsizing of the valve can be dispensed with due to the above described cooperation between the displacement unit 101 and the stent 105. The stent 105 may thus be more robust and less complex, and therefore more reliable in sustaining the desired function of the valve over time. Prior art implants may on the contrary require reoccurring adjustments, due to a complex interplay between several moving parts in order to provide downsizing of the annulus. The annuloplasty device 100 provides also for reducing the risk of damaging the CS as the downsizing may be provided by an atraumatically shaped displacement unit 101, instead of the stent 105 which may have retention units 110 as described further below. The risk of tearing of the tissue in the CS with such retention units may thus be reduced.

As described, the distance (L) between the proximal expandable portion 102 and the distal anchoring portion 103 in the longitudinal direction 104 may decrease to a reduced distance (L') when the displacement unit 101 is transferred from the delivery state to the activated state. The proximal expandable portion 102 and the distal anchoring portion 103 may be connected to different sheaths or wires, that may be independently movable in the longitudinal direction 104 to provide for varying the distance (L) as illustrated in Figs. 3a-b.

The proximal expandable portion 102 is connected to a sheath 107 and is configured to be expanded in a radial direction (R), perpendicular to the longitudinal direction 104, by pushing a proximal portion 108 of the sheath 107 towards the distal anchoring portion 103, as indicated in Fig. 2b (see arrow adjacent sheath 107). This provides for a facilitated deployment of the expandable portion 102 to the expanded configuration.

As the displacement unit 101 is positioned with a separation (L') between the proximal expandable portion 102 and the distal anchoring portion 103 that provides the desired downsizing of the valve, the stent 105 is movable into position over the displacement unit 101 for positioning and anchoring into the CS. The annuloplasty device 100 comprises a catheter 109 to enclose the stent 105 and position the stent 105 relative the displacement unit 101 in the longitudinal direction 104, as schematically illustrated in Figs. 4a-b, and Figs. 5c-f. Fig. 4a show an example where the catheter 109 is first advanced over the displacement unit 101, before the stent 105 is pushed forward inside the catheter 109 by a delivery device 106. It is conceivable however that the stent 105 may be advanced over the displacement unit 101 at the same time as the catheter 109. The stent 105 may be ejectable from, and retrievable into, the catheter 109 by the aforementioned delivery device 106. Fig. 4c is a schematic illustration where the catheter 109 has been withdrawn to expose the stent 105 over the displacement unit 101. Fig. 4d is a schematic illustration where the displacement unit 101 has been withdrawn through the stent 105. The proximal expandable portion 102 is collapsed and the stent 105 may be released from the delivery device 106.

The catheter 109 is thus be movable inside the sheath 107 in the longitudinal direction 104. The stent 105 may thus be positioned at the desired position over the displacement unit 101 while the proximal expandable portion 102, being connected to the sheath 107, is expanded and anchored against the entrance of the CS.

The catheter 109 is thus be movable over the displacement unit 101, and inside said sheath 107, in the longitudinal direction 104, in the aforementioned activated state. This provides for an efficient and reliable positioning and deployment of the stent 105 in the CS while the amount of downsizing of the annulus is effectively controlled by the displacement unit 101.

The stent 105 may be reversibly expandable in the radial direction (R) in the activated state. Thus, once expanded, e.g. as illustrated in the (partly) expanded state in Fig. 5g, the stent 105 may be collapsed and retrieved again if needed in order to reposition or replacing the stent 105. For retrieval, the stent 105 may be pulled into the catheter 109 by withdrawing delivery device 106 relative the catheter 109, thereby forcing the stent 105 into the confinement of the catheter 109. The stent 105 may be self-expandable such that it strives towards an expanded diameter once being release from the catheter 109. The stent 105 may in such case be formed from a shape memory material which has been heat set in an expanded diameter configuration where the diameter is larger than the diameter of the CS. The stent 105 may be compressed and inserted into the catheter 105 before being ejected in the CS where the stent 105 will strive towards the heat set shape and thus press against the tissue walls inside the CS. It is also conceivable that the stent 105 may be actively expanded by e.g. a balloon catheter pushing inside the stent 105 to expand its diameter.

The stent 105 may comprise retention units 110 to anchor the stent 105 in the CS, as schematically illustrated in Figs. 4c-d, 5g-l, and 6b. Thus, once the displacement unit 101 has contracted the tissue around the CS to re-shape the annulus, by shortening from a length (L) to a reduced length (L') (Figs. 3a-b), the shape of the remodelled tissue may be retained by the stent 105 which is anchored into the tissue. Having retention units 110 provides for an effective and reliable anchoring of the stent 105 into the tissue. The stent 105 may extend with an uninterrupted length in abutment with the tissue wall of the CS, along the majority of the length of the CS. This provides for a reliable retention of the re-shaped annulus over time, in particular when having retention units 110 extending essentially along the full length of the stent 105. The stent 105 may have a length that corresponds essentially to the length of the CS, as schematically indicated in Figs. 5k-l. Retention units 110 may be provided along the length of the stent 105. This further provides for particularly reliable retention of the re-shaped annulus over time.

The retention units 110 may be arranged on a surface section 111 of the stent 105 being adapted to be arranged towards the annulus when the stent 105 is in the CS, as schematically indicated in the top-down view of Fig 6b in conjunction with Fig. 4e showing a cross-section of the stent 105 in one example. The retention units 110 may thus be arranged at a determined circle sector (v) on the surface of the stent as indicated in Fig. 4e. Having the retention units 110 arranged in a direction towards the annulus provides for an effective retention of the tissue along the corresponding segment of the CS and a reliable retention of the modified annulus shape. The stent 105 may comprise at least one radiopaque marker (not shown) in one example. This provides for a facilitated orienting of the stent 105 in relation to the direction of the annulus.

It is conceivable that a plurality of retention units 110 may be arranged around the circumference of the stent 105 in one example. Retention units 110 may thus be arranged at a plurality of circle sectors (v) along the circumference of the stent 105. This may be advantageous in some applications where an increased retention force is desirable.

The retention units 110 may be shaped to pierce into tissue in the CS and thereby provide a retention force into the tissue. The retention units 110 may be formed from the material of the stent 105. The retention units 110 may thus be integrated with the stent 105. The retention units 110 may thus be cut as respective elongated structures with piercing tips within the structural framework of the stent 105. Forming the retention units 110 as integrated structures of the framework of the stent 105 provides for robust and strong retention units 110 and a minimized risk of dislocations or deformations thereof over time. An overall robust and reliable fixation mechanism is thus provided. The retention units 110 may be formed by different cutting techniques such as by laser cutting techniques.

The retention units 110 may be resiliently moveable from a retracted state to an expanded state. Thus, the retention units 110 may be flexible to bend from the expanded state to the retracted state when arranged inside the catheter 109, and to expand from the retracted state to the expanded state when released from the catheter 109. This provides for a facilitated delivery of the stent 105 through the catheter 109, while allowing for expansion and anchoring of the retention units 110 into the tissue once deployed from the confinement of the catheter 109. The retention units 110 may thus be heat-set to assume a defined expanded shape, as indicated in e.g. Figs. 4d-e. The expanded shape may thus correspond to a relaxed state of the retention unit 110 where the latter is not acted upon by external forces. The retention unit 110 may thus have a bias towards the expanded shape, by striving towards the relaxed expanded state, when released from the catheter 109.

The retention units 110 may be are aligned essentially flush with an outer diameter of the stent in the retracted state. This provides further for a facilitated delivery of the stent 105 through the catheter 109, as friction between the retention units 110 and the inside lumen of the catheter 109 may be reduced. Further, a compact cross-section is provided and a minimized risk of abrasion and damage to the catheter 109.

In one example the retention units 110 may comprise a shape-memory material, where activation of the shape-memory material causes the retention units 110 to transfer from the retracted state to the expanded state. For example, the shape-memory material may be temperature activated, so that the retention units 110 move towards the expanded state when subject to heating to the body temperature. This provides for an advantageous deployment of the retention units 110 in some applications.

The distal anchoring portion 103 may comprise an inflatable unit, such as a balloon, which is expandable in the radial direction (R). This provides for efficient and non-traumatic fixation of the distal end of the displacement unit 101, which in combination with the efficient anchoring against the wall of the CS by the proximal portion 102, allows for an efficient transfer of a contracting force of the proximal and distal portions 102, 103, towards each other. This allows for an effective modification of the radius of curvature of the CS to facilitate modifying the shape of the valve annulus. The annuloplasty device 100 may comprise an inflation lumen (not shown) connected to the inflatable unit and being configured to deliver an inflation medium to the inflatable unit.

The length of inflatable unit 103 may be adapted to varying anatomies. The length of the inflatable unit 103 may be chosen so that it does not block vessels connecting to the CS, e.g. if the inflatable unit 103 is anchored further into the CS, such as towards the great cardiac vein/left coronary vein. The length of the inflatable unit 103 may also be adapted so that it may be effectively anchored behind the bend or "corner" of the CS as it transitions into the great cardiac vein/left coronary vein. The length of the inflatable unit 103 may be sufficiently short to facilitate such anchoring and avoid slipping out of this bend or "corner" of the CS.

The proximal expandable portion 102 may comprise expandable bows or ribs 112. The sheath 107 may be pushed in relation to a distal portion 114 attached distally to the bows or ribs 112. The compressive force between the distal portion 114 and the proximal portion 108 may thus push the bows 112 radially outwards. It is conceivable however that the bows 112 may comprise a shape-memory material having a tendency to assume the expanded configuration in its relaxed state, and that the bows may be confined in an outer sheath (not shown) being pulled back so that the bows 112 spring into the expanded configuration.

Having expandable bows 112 provides for to further lessen the risk of damaging the tissue at the entrance of the CS, since a soft apposition against the tissue may be provided, in absence of sharp edges or kinks. The bows 112 may extend in the longitudinal direction 104 which facilitates a symmetric engagement against the tissue wall, with an even transfer of force around the entrance to the CS, hence allowing for a robust anchoring. The longitudinal extension of the bows 112 also provides for facilitated expansion of the bows 112 by applying a force to the bows 112 in the longitudinal direction 104. A plurality of bows 112 may be arranged circumferentially so that a force may be applied symmetrically and evenly around the tissue wall.

The proximal expandable portion 102 may comprise elongated ribs 112 formed in the sheath 107 by elongated cuts 113 in the sheath 107, extending in the longitudinal direction 104, as schematically illustrated in Fig. 2a. The ribs 112 may be foldable to expand in the radial direction (R). This provides for a simple and robust construction. The ribs or bows 112 may thus be formed from the same material as the sheath 107. The mentioned material may be a soft flexible material which is non-traumatic to tissue. In the collapsed configuration seen in Fig. 2a, the ribs 112, i.e. the soon to be expanded ribs 112, extend in the longitudinal direction 104, and provides for a compact radial profile. The ribs or bows 112 may be placed equidistantly around a circumference of the sheath 107. As elucidated above, this may provide for an even distribution of the anchoring force.

The maximum expanded diameter (D) of the proximal expandable portion 102 may be at least three times the diameter of the CS. In some examples the ratio of the maximum expanded diameter (D) of the proximal expandable portion 102 to the diameter of the CS is in the range 3-5. In some examples the aforementioned ratio may be in the range 3.5 - 4.5, which provides for a particular advantageous anchoring of the proximal expandable portion 102, while maintaining a compact and easy to use annuloplasty device 100.

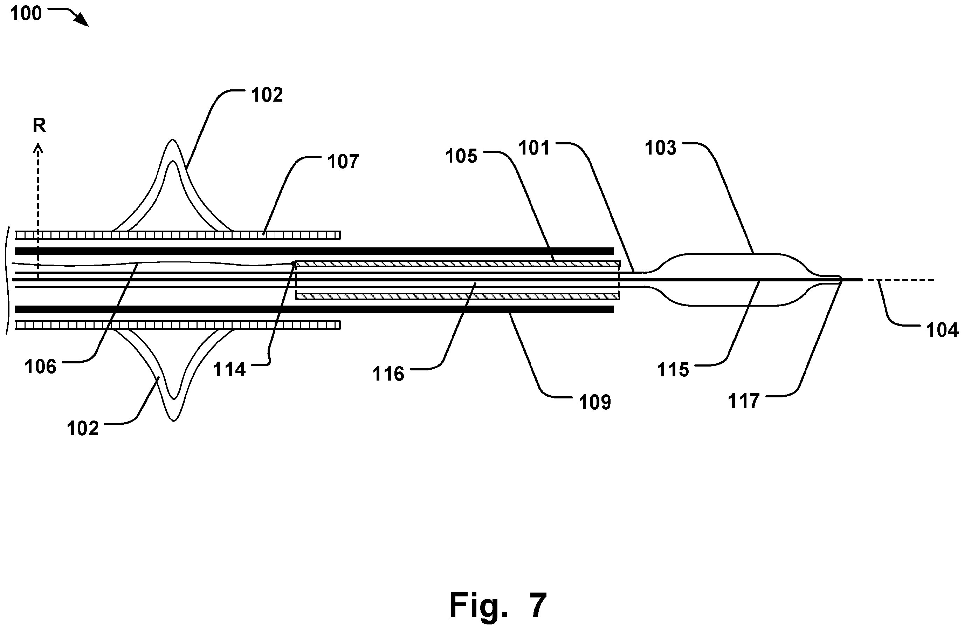

The annuloplasty device 100 may comprise a guide wire 115 arranged to extend inside a lumen 116 of the displacement unit 101 and to exit the lumen 116 at a distal opening 117 of the displacement unit 101, as schematically illustrated in Fig. 7. The guide wire 115 may be inserted into the CS and the displacement unit 101 may subsequently be advanced over the guide wire 115 for positioning in the CS. This provides for a facilitated positioning of the displacement unit 101.

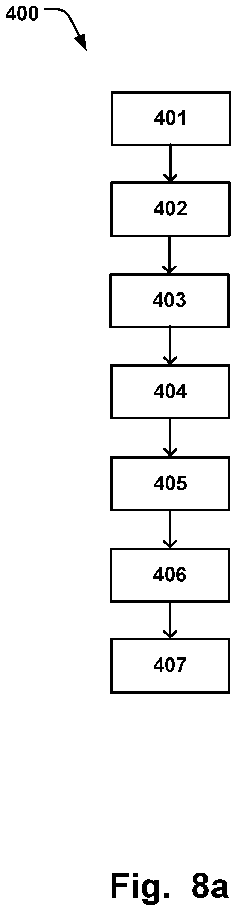

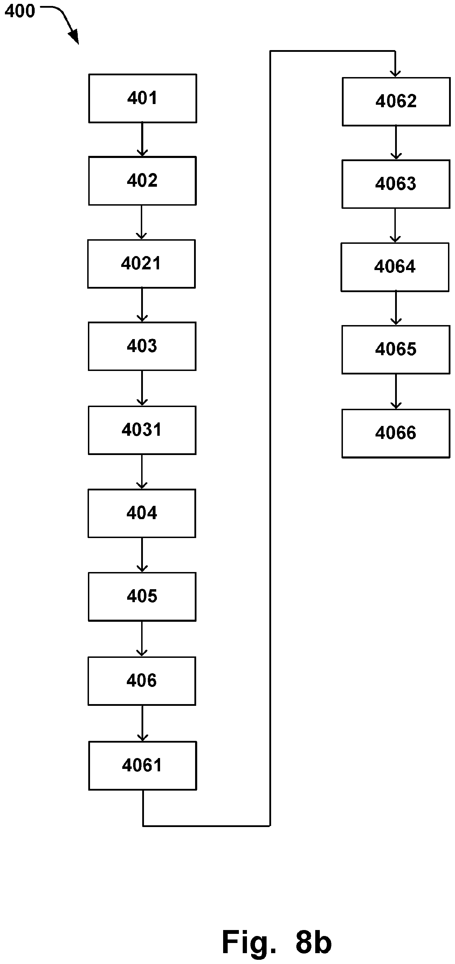

Fig. 8a illustrates a method 400 for treating a defective mitral valve. The order in which the steps of the method 400 are illustrated should not be construed as limiting and it is conceivable that the order in which the steps of the method 400 is carried out may be varied. The method 400 comprises inserting 401 a flexible and removable elongate displacement unit 101 in a delivery state into a coronary sinus CS adjacent said valve; positioning 402 a proximal reversibly expandable portion 102 against a tissue wall at the entrance of said CS (Fig. 5a); anchoring 403 a distal anchoring portion 103 inside the CS (Fig. 5b); activating 404 the displacement unit 101 in an activated state whereby the distal anchoring portion 103 is moved in a longitudinal direction 104 of the displacement unit 101 to reduce a distance (L) between the distal anchoring portion 103 and the proximal expandable portion 102 such that the shape of the annulus is modified to a modified shape. The method 400 further comprises advancing 405 a stent 105 through the proximal expandable portion 102 and over the displacement unit 101 into the CS (Figs. 5e-f); anchoring 406 the stent 105 in the CS to retain the modified shape of the annulus (Figs. 5g-i); withdrawing 407 the displacement unit 101 through the stent 105 to remove 408 the displacement unit 101 after temporary activation in the activated state (Figs. 5j-l, 6b). The method 400 thus provides for the advantageous benefits as described above in relation to the annuloplasty device 100 and Figs. 1 - 7. The method 400 provides for an improved annuloplasty procedure with an increase in the degree of control of the downsizing procedure, while ensuring secure anchoring of the stent 105 and minimal risk of damage to the CS. Advancing the stent 105 over the displacement unit 101 into the CS while the proximal expandable portion 102 is expanded at the outside of the CS and the displacement unit 101 is in the activated state provides for a secure positioning and fixation of the stent 105 when the valve is already re-shaped by the displacement unit 101. A particularly robust and reliable annuloplasty procedure is thus provided.

Fig. 6b illustrates another flow chart of a method 400 for treating a defective mitral valve. The order in which the steps of the method 400 are illustrated should not be construed as limiting and it is conceivable that the order in which the steps of the method 400 is carried out may be varied.

The distal anchoring portion 103 may comprise an inflatable unit, also denoted with reference numeral 103. Anchoring the distal anchoring portion 103 may comprise inflating 4031 the inflatable unit 103 in the coronary sinus, and/or in the great cardiac vein and/or, in the anterior interventricular branch or vein, and/or in the posterior vein and/or in the posterior ventricular vein of the heart.

The proximal expandable portion 102 may be connected to a sheath 107. Positioning the proximal expandable portion 102 may comprise pushing 4021 a proximal portion 108 of the sheath 107 towards the distal anchoring portion 103 to expand the proximal expandable portion 102 in a radial direction (R).

Anchoring the stent 105 may comprise withdrawing 4061 a catheter 109 enclosing the stent 105 and expanding 4062 the stent 105 in a radial direction (R) being perpendicular to the longitudinal direction 104, as schematically illustrated in Figs. 5g-h.

The catheter 109 may be is movable through the proximal expandable portion 102 and over the displacement unit 101 in the longitudinal direction 104, providing for the advantageous effects as described above.

Anchoring the stent 105 may comprise anchoring 4063 retention units 110 of the stent 105 into the CS to retain the modified shape of the annulus when the displacement unit 101 is withdrawn, as schematically illustrated in Figs. 5g-l.

Anchoring the retention units 110 may comprise anchoring 4064 the retention units 110 in a tissue wall of the CS in the direction of the annulus.

The method 400 may comprise advancing 4065 the catheter 109 over the stent 105 to disengage the stent 105 from the CS for repositioning or removal of the stent 105 from the CS.

Anchoring the stent 105 may comprise releasing 4066 the stent 105 from a delivery device 106 movably arranged inside the catheter 109 (Fig. 5k).

The present invention has been described above with reference to specific embodiments.

More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. An annuloplasty device (100) for treating a defective mitral valve having an annulus, comprising
a removable and flexible elongate displacement unit (101) for temporary insertion into a coronary sinus (CS) adjacent the valve, wherein the displacement unit has a delivery state for delivery into the CS, and an activated state to which the displacement unit is temporarily and reversibly transferable from said delivery state,
a proximal reversibly expandable portion (102) being reversibly foldable to an expanded state for positioning against a tissue wall at the entrance of the CS,
wherein the displacement unit comprises a distal anchoring portion (103) being movable in relation to the proximal expandable portion in a longitudinal direction (104) of the displacement unit to said activated state in which the shape of the annulus is modified to a modified shape when inserted in the CS,
a stent (105) arranged around the displacement unit and being movable relative the displacement unit along the longitudinal direction for insertion into the CS, and
wherein the stent is releasably connected to a delivery device (106) and arranged radially between the displacement unit and the proximal expandable portion in a radial direction (R), the radial direction (R) being perpendicular to the longitudinal direction,
**characterized in that** the proximal expandable portion (102) is connected to a sheath (107) and is configured to be expanded in the radial direction (R) by pushing a proximal portion (108) of the sheath towards the distal anchoring portion, the annuloplasty device comprising
a catheter (109) to enclose the stent and position the stent relative the displacement unit in the longitudinal direction, wherein the stent is ejectable from, and retrievable into, the catheter by the delivery device,
wherein the catheter is movable over the displacement unit and inside said sheath in the longitudinal direction, in said activated state.

2. Annuloplasty device according to claim 1, wherein the stent is reversibly expandable in the radial direction (R) in said activated state.

3. Annuloplasty device according to claim 1 or 2, wherein the stent comprises retention units (110) to anchor the stent in the CS.

4. Annuloplasty device according to claim 3, wherein the retention units are arranged on a defined surface section (111) of the stent adapted to be arranged towards the annulus when the stent is in the CS.

5. Annuloplasty device according to claim 3, wherein a plurality of retention units are arranged around a circumference of the stent.

6. Annuloplasty device according to any of claims 3 - 5, wherein the retention units are resiliently moveable from a retracted state to an expanded state, whereby the retention units are flexible to
bend from the expanded state to the retracted state when arranged inside the catheter, and
expand from the retracted state to the expanded state when released from the catheter.

7. Annuloplasty device according to claim 6, wherein the retention units are aligned essentially flush with an outer diameter of the stent in the retracted state.

8. Annuloplasty device according to any of claims 1 - 7, wherein a distance (L) between the proximal expandable portion and the distal anchoring portion in the longitudinal direction decreases to a reduced distance (L') when the displacement unit is transferred from the delivery state to the activated state.

9. Annuloplasty device according to any of claims 1 - 8, wherein the proximal expandable portion comprise elongated ribs (112) formed in a sheath (107) by elongated cuts (113) in the sheath, extending in the longitudinal direction, the ribs being foldable to expand in the radial direction (R).

10. Annuloplasty device according to any of claims 1-9, wherein the distal anchoring portion comprises an inflatable unit being expandable in the radial direction (R).

11. Annuloplasty device according to any of claims 1 - 10, comprising a guide wire (115) arranged to extend inside a lumen (116) of the displacement unit and to exit the lumen at a distal opening (117) of the displacement unit.

## Patentansprüche

1. Anuloplastievorrichtung (100) zur Behandlung einer defekten Mitralklappe mit einem Anulus, umfassend
eine entfernbare und flexible längliche Verschiebeeinheit (101) zum vorübergehenden Einführen in einen der Klappe benachbarten Koronarvenensinus (CS), wobei die Verschiebeeinheit einen Zuführzustand zum Zuführen in den CS und einen aktivierten Zustand hat, in den die Verschiebeeinheit aus dem Zuführzustand vorübergehend und reversibel transferierbar ist,
einen proximalen reversibel expandierbaren Abschnitt (102), der reversibel in einen expandierten Zustand faltbar ist, um gegen eine Gewebewand an dem Eingang des CS positioniert zu werden,
wobei die Verschiebeeinheit einen distalen Verankerungsabschnitt (103) umfasst, der bezüglich des proximalen expandierbaren Abschnitts in eine Längsrichtung (104) der Verschiebeeinheit zu dem aktivierten Zustand bewegbar ist, in dem die Form des Anulus zu einer modifizierten Form modifiziert ist, wenn er in den CS eingeführt ist,
einen Stent (105), der um die Verschiebeeinheit herum angeordnet und bezüglich der Verschiebeeinheit entlang der Längsrichtung zum Einführen in den CS bewegbar ist, und
wobei der Stent freigebbar mit einer Zuführungsvorrichtung (106) verbunden und radial zwischen der Verschiebeeinheit und dem proximalen expandierbaren Abschnitt in einer radialen Richtung (R) angeordnet ist, wobei die radiale Richtung (R) senkrecht zu der Längsrichtung verläuft,
**dadurch gekennzeichnet, dass** der proximale expandierbare Abschnitt (102) mit einer Hülse (107) verbunden und dazu ausgestaltet ist, in der radialen Richtung (R) expandiert zu werden, indem ein proximaler Abschnitt (108) der Hülse zu dem distalen Verankerungsabschnitt hin geschoben wird, wobei die Anuloplastievorrichtung
einen Katheter (109) umfasst, um den Stent zu umschließen und den Stent bezüglich der Verschiebeeinheit in der Längsrichtung zu positionieren, wobei der Stent mittels der Zuführungsvorrichtung aus dem Katheter ausstoßbar und in diesen zurückziehbar ist,
wobei der Katheter in dem aktivierten Zustand über die Verschiebeeinheit und in der Hülse in der Längsrichtung bewegbar ist.

2. Anuloplastievorrichtung nach Anspruch 1, wobei der Stent in dem aktivierten Zustand in der radialen Richtung (R) reversibel expandierbar ist.

3. Anuloplastievorrichtung nach Anspruch 1 oder 2, wobei der Stent Halteeinheiten (110) umfasst, um den Stent in dem CS zu verankern.

4. Anuloplastievorrichtung nach Anspruch 3, wobei die Halteeinheiten auf einer definierten Oberflächensektion (111) des Stents angeordnet und dazu ausgeführt sind, zu dem Anulus hin angeordnet zu sein, wenn der Stent in dem CS ist.

5. Anuloplastievorrichtung nach Anspruch 3, wobei eine Vielzahl von Halteeinheiten um einen Umfang des Stents herum angeordnet sind.

6. Anuloplastievorrichtung nach einem der Ansprüche 3 - 5, wobei die Halteeinheiten federnd aus einem zurückgezogenen Zustand zu einem expandierten Zustand bewegbar sind, wodurch die Halteeinheiten flexibel sind,
um sich aus dem expandierten Zustand in den zurückgezogenen Zustand zu biegen, wenn sie in dem Katheter angeordnet sind, und
um aus dem zurückgezogenen Zustand in den expandierten Zustand zu expandieren, wenn sie aus dem Katheter freigegeben werden.

7. Anuloplastievorrichtung nach Anspruch 6, wobei die Halteeinheiten in dem zurückgezogenen Zustand im Wesentlichen bündig auf einen Außendurchmesser des Stents ausgerichtet sind.

8. Anuloplastievorrichtung nach einem der Ansprüche 1 - 7, wobei ein Abstand (L) zwischen dem proximalen expandierbaren Abschnitt und dem distalen Verankerungsabschnitt in der Längsrichtung zu einem reduzierten Abstand (L') abnimmt, wenn die Verschiebeeinheit aus dem Zuführzustand in den aktivierten Zustand transferiert wird.

9. Anuloplastievorrichtung nach einem der Ansprüche 1 - 8, wobei der proximale expandierbare Abschnitt Längsrippen (112) umfasst, die durch Längsschnitte (113) in der Hülse in einer Hülse (107) ausgebildet sind, die sich in der Längsrichtung erstrecken, wobei die Rippen zur Expandierung in der radialen Richtung (R) faltbar sind.

10. Anuloplastievorrichtung nach einem der Ansprüche 1 - 9, wobei der distale Verankerungsabschnitt eine inflatierbare Einheit umfasst, die in der radialen Richtung (R) expandierbar ist.

11. Anuloplastievorrichtung nach einem der Ansprüche 1 - 10, umfassend einen Führungsdraht (115), der so angeordnet ist, dass er sich in einem Lumen (116) der Verschiebeeinheit erstreckt und an einer distalen Öffnung (117) der Verschiebeeinheit aus dem Lumen austritt.

## Revendications

1. Dispositif d'annuloplastie (100) permettant de traiter une valve mitrale défectueuse comportant un anneau, comprenant
une unité de déplacement allongée, amovible et flexible (101) destinée à être insérée temporairement dans un sinus coronaire (CS) adjacent à la valvule, dans lequel l'unité de déplacement présente un état de pose pour une pose dans le CS, et un état activé dans lequel l'unité de déplacement est temporairement et réversiblement transférable à partir dudit état de pose,
une partie proximale déployable de manière réversible (102) pouvant être repliée réversiblement vers un état déployé pour être positionnée contre une paroi tissulaire à l'entrée du CS,
dans lequel l'unité de déplacement comprend une partie d'ancrage distale (103) étant mobile par rapport à la partie déployable proximale dans une direction longitudinale (104) de l'unité de déplacement vers ledit état activé dans lequel la forme de l'anneau est modifiée vers une forme modifiée lorsqu'elle est insérée dans le CS,
une endoprothèse (105) agencée autour de l'unité de déplacement et pouvant être déplacée par rapport à l'unité de déplacement le long de la direction longitudinale pour être insérée dans le CS, et
dans lequel l'endoprothèse est reliée de manière libérable à un dispositif de pose (106) et agencée radialement entre l'unité de déplacement et la partie déployable proximale dans une direction radiale (R), la direction radiale (R) étant perpendiculaire à la direction longitudinale,
**caractérisé en ce que** la partie déployable proximale (102) est reliée à une gaine (107) et est conçue pour être déployée dans la direction radiale (R) en poussant une partie proximale (108) de la gaine vers la partie d'ancrage distale, le dispositif d'annuloplastie comprenant
un cathéter (109) pour enfermer l'endoprothèse et positionner l'endoprothèse par rapport à l'unité de déplacement dans la direction longitudinale, dans lequel l'endoprothèse est éjectable de, et récupérable dans, le cathéter par le dispositif de pose,
dans lequel le cathéter est mobile sur l'unité de déplacement et à l'intérieur de ladite gaine dans la direction longitudinale, dans ledit état activé.

2. Dispositif d'annuloplastie selon la revendication 1, dans lequel l'endoprothèse est déployable de manière réversible dans la direction radiale (R) dans ledit état activé.

3. Dispositif d'annuloplastie selon la revendication 1 ou 2, dans lequel l'endoprothèse comprend des unités de retenue (110) pour ancrer l'endoprothèse dans le CS.

4. Dispositif d'annuloplastie selon la revendication 3, dans lequel les unités de retenue sont agencées sur une section de surface définie (111) de l'endoprothèse conçue pour être agencée vers l'anneau lorsque l'endoprothèse est dans le CS.

5. Dispositif d'annuloplastie selon la revendication 3, dans lequel une pluralité d'unités de retenue est agencée autour d'une circonférence de l'endoprothèse.

6. Dispositif d'annuloplastie selon l'une quelconque des revendications 3 à 5, dans lequel les unités de retenue sont mobiles de manière élastique d'un état rétracté à un état déployé, moyennant quoi les unités de retenue sont flexibles pour
se plier de l'état déployé à l'état rétracté lorsqu'elles sont agencées à l'intérieur du cathéter, et
se déployer de l'état rétracté à l'état déployé lorsqu'elles sont libérées du cathéter.

7. Dispositif d'annuloplastie selon la revendication 6, dans lequel les unités de retenue sont alignées essentiellement au ras d'un diamètre externe de l'endoprothèse à l'état rétracté.

8. Dispositif d'annuloplastie selon l'une quelconque des revendications 1 à 7, dans lequel une distance (L) entre la partie déployable proximale et la partie d'ancrage distale dans la direction longitudinale diminue jusqu'à une distance réduite (L') lorsque l'unité de déplacement est transférée de l'état de pose à l'état activé.

9. Dispositif d'annuloplastie selon l'une quelconque des revendications 1 à 8, dans lequel la partie déployable proximale comprend des nervures allongées (112) formées dans une gaine (107) par des découpes allongées (113) dans la gaine, s'étendant dans la direction longitudinale, les nervures étant pliables pour se déployer dans la direction radiale (R).

10. Dispositif d'annuloplastie selon l'une quelconque des revendications 1 à 9, dans lequel la partie d'ancrage distale comprend une unité gonflable étant déployable dans la direction radiale (R).

11. Dispositif d'annuloplastie selon l'une quelconque des revendications 1 à 10, comprenant un fil-guide (115) conçu pour s'étendre à l'intérieur d'une lumière (116) de l'unité de déplacement et pour sortir de la lumière au niveau d'une ouverture distale (117) de l'unité de déplacement.
